Europäisches Patentamt

European Patent Office

Office européen des brevets

(19)

(11) Veröffentlichungsnummer: **0 216 189**
**B1**

(12) **EUROPÄISCHE PATENTSCHRIFT**

(45) Veröffentlichungstag der Patentschrift:
02.11.89

(51) Int. Cl.⁴: **A61K 7/16**

(21) Anmeldenummer: **86111944.4**

(22) Anmeldetag: **29.08.86**

(54) **Mittel zur oralen Hygiene.**

(30) Priorität: **14.09.85 DE 3532860**

(43) Veröffentlichungstag der Anmeldung:
**01.04.87 Patentblatt 87/14**

(45) Bekanntmachung des Hinweises auf die Patenterteilung:
**02.11.89 Patentblatt 89/44**

(84) Benannte Vertragsstaaten:
**AT BE CH DE FR GB IT LI LU NL SE**

(56) Entgegenhaltungen:
**EP-A- 0 038 867**
**EP-A- 0 161 899**
**FR-A- 2 044 388**

(73) Patentinhaber: **Blendax GmbH, Rheinallee 88,
D-6500 Mainz(DE)**

(72) Erfinder: **Wagner, Helmar R., Römerstrasse 62,
D-6100 Darmstadt-Arheilgen(DE)**

ACTORUM AG

## Beschreibung

Die vorliegende Erfindung betrifft ein Mittel zur oralen Hygiene, insbesondere ein Zahn- und Mundpflegemittel mit einer die Bildung von Zahnbelag hemmenden Wirksamkeit, das als belagshemmende Wirkstoffe ein Gemisch aus Kupferverbindungen und 2,4,4'-Trichlor-2'-hydroxydiphenylether enthält.

Es ist bekannt, daß das Kupfer-Kation eine die Bildung von Zahnbelag hemmende Wirkung ausübt, wenn entsprechende Lösungen von Kupferverbindungen topisch mit den Zähnen in Berührung gebracht werden (AADR-Abstracts 1979, No. 117; Caries Research, Vol. 18, 1984, S. 434 - 439).

Diese Kupferionen liefernden Verbindungen weisen die Nachteile, wie sie für andere belagshemmende Verbindungen auf Basis antimikrobieller Stoffe wie beispielsweise quaternären Ammoniumverbindungen oder Chlorhexidinsalzen bekannt geworden sind, insbesondere die Verfärbung der Zähne bei Langzeitanwendung, nicht auf.

Ihrer Anwendung in Zahnpasten und anderen, weitere Wirk- und Hilfsstoffe enthaltenen Mitteln stand jedoch bislang entgegen, daß sie einerseits durch verschiedene dieser Stoffe inaktiviert wurden oder nicht bei jedem wünschenswerten pH-Werkt ihre Aktivität aufrechterhielten.

In den EP-A 38 867 und 38 868 sind bereits Zahnpasta-Zusammensetzungen beschrieben, die Kupferverbindungen auch in aktiver Form enthalten. Dies wird gewährleistet durch die optimale Auswahl des jeweiligen Poliermittels.

Obwohl diese Zusammensetzungen sich als prinzipiell aktiv gegen Zahnbelag erwiesen haben, ist es doch wünschenswert, in der Auswahl der Poliermittel und des pH-Wertes nicht auf bestimmte Komponenten bzw. Bereiche eingeschränkt zu sein.

Es bestand daher das Bedürfnis, ein Kupferionen lieferndes Zahn- und Mundpflegemittel mit belagshemmender Wirkung zu entwickeln, das beliebig einsetzbar ist und durch in solchen Mitteln üblicherweise vorhandene andere Stoffe in seiner Wirkung nicht negativ beeinflußt wird, sondern sogar noch eine gegenüber den Einzelkomponenten verbesserte Wirksamkeit aufweist.

Diese komplexe Aufgabe wurde überraschenderweise dadurch gelöst, daß man einem Kupferionen enthaltenden Zahnpflegemittel 2,4,4'-Trichlor-2'-hydroxydiphenylether, vorzugsweise in einer Menge von etwa 0,02, insbesondere 0,05 bis etwa 1,5, insbesondere 0,1 Gew.-%, berechnet auf die Gesamtzusammensetzung des Mittels, zusetzt.

Die Menge an Kupferverbindungen, die in den erfindungsgemäßen Zahnpflegemitteln zum Einsatz gelangt, sollte so bemessen sein, daß etwa 0,001 bis etwa 5 Gew.-% Cu, berechnet auf die gesamte Zahnpasta, anwesend sind.

Ein bevorzugter Mengenbereich liegt bei 0,05 bis 1,5%, insbesondere bei etwa 0,5% Cu.

Als Kupferionen liefernde Verbindungen sind prinzipiell alle toxikologisch unbedenklichen, schleimhautverträglichen, auch nur einigermaßen wasserlöslichen Kupferverbindungen geeignet.

Von den anorganischen Salzen seien beispielhaft genannt:

Kupferchlorid, $CuCl_2$, und dessen Dihydrat; Kupferfluorid, $CuF_2$, und dessen Dihydrat; Kupferfluosilikat, $CuSiF_6$, und dessen Hexahydrat; Kupfersulfat, $CuSO_4$, und dessen Pentahydrat; Kupfernitrat bzw. dessen Tri- und Hexahydrat sowie auch ausgefallenere Kupfersalze wie Bromide, Bromate, Chlorate, Jodate, Fluorphosphate.

Bevorzugte Kupfersalze organischer Säuren sind das Acetat, Formiat, Benzoat, Citrat, Tartrat, Lactat, Malat, Mandelat, Sorbat, Pantothenat, Gluconat, Phytat, Glycerophosphat, Cinnamat, Butyrat, Propionat, Laurat, Oxalat, Salicylat.

Geeignet sind auch die Kupfersalze von Aminosäuren wie Glycinat oder Glutamat. Ein besonders bevorzugtes Aminosäuresalz ist das Kupferaspartat.

Aus den im folgenden beschriebenen Vergleichsversuchen ergibt sich die überlegene Wirksamkeit einer Kupfersulfat (0,05 Gew.-%, berechnet auf Kupfer) und 2,4,4'-Trichlor-2'-hydroxydiphenylether enthaltenen Zahnpasta gegenüber Zahnpasten mit einem Gehalt an Kupfersulfat bzw. 2,4,4'-Trichlor-2'-hydroxyphenylether sowie unbehandelten Kontrollgruppen:

20 Tage alte Osborne-Mendel-Ratten wurden in 4 Gruppen mit je 16 Versuchstieren aufgeteilt und erhielten die Plaque-Standard-Lost 2000 F.

Zu Versuchsbeginn wird von jedem Versuchstier der Plaque-Ausgangs-Status erstellt. Anschließend werden die Tiere mit 0,1 ml einer standardisierten Bakteriensuspension von Actinomyces viscosus OMZ 105 geimpft. Eine Nachimpfung erfolgt jeweils in einwöchigem Abstand.

Die Behandlung der Versuchstiere mit den zu untersuchenden Präparaten beginnt am 23. Lebenstag, je zweimal täglich werden mit einer Spritze 0,1 ml Testpräparat/Versuchstier appliziert. Nach 4 Wochen wurden die Tiere getötet und die Plaquebildung an den beiden ersten Bukkalflächen und den ersten 4 Lingualflachen der Molaren 1 und 2 im Oberkiefer beurteilt; d.h., 12 Begutachtungsflächen/Tier.

Die Bewertung erfolgt nach Anfärbung mit Erythrosin-Lösung nach dem folgenden Schema:

0: Keine Plaque
1: Bis zu 1/3 der Fläche mit Plaque überzogen.
2: Bis zu 2/3 der Fläche mit Plaque überzogen.
3: Mehr als 2/3 der Fläche mit Plaque überzogen.

Die Punktzahl wird addiert und ein Mittelwert($\overline{x}$) gebildet.

| Ergebnis | | |
|----------|--------|---------|
| Gruppe | $\overline{x}$ | |
| 1 | 18,56 | +/– 1,27 |
| 2 | 12,73 | +/– 1,65 |
| 3 | 18,79 | +/– 1,24 |
| 4 | 23,00 | +/– 1,68 |

Zusammensetzung der Zahnpasten

Gruppe 1: Entsprechend Beispiel A, jedoch ohne Kupfersulfat.
Gruppe 2: Entsprechend Beispiel A.
Gruppe 3: Entsprechend Beispiel A, jedoch ohne 2,4,4'-Trichlor-4'-hydroxydiphenylether.
Gruppe 4: Unbehandelte Kontrolle.

Diese Ergebnisse beweisen die Überlegenheit der erfindungsgemäßen synergistischen Kombination aus Kupferverbindungen und 2,4,4'-Trichlor-2'-hydroxydiphenylether.

| Beispiel A | |
|-----------|---|
| α-Aluminiumoxidtrihydrat (Korngrößenverteilung etwa 1–15 μm) | 58,5 (Gew.-%) |
| Sorbitlösung (70%) | 25,5 |
| Xanthum-Gum | 0,6 |
| Natriummonofluorphosphat | 0,8 |
| Saccharin-Natrium | 0,1 |
| Konservierungsmittel | 0,3 |
| Natriumlaurylsulfat | 0,4 |
| Aromagemisch | 0,1 |
| Kupfersulfat · 5 $H_2O$ | 0,2 |
| 2,4,4'-Trichlor-2'-hydroxydiphenylether | 0,1 |
| Wasser | ad 100,0 |

Die erfindungsgemäßen Mittel zur oralen Hygiene können in jeder beliebigen Applikationsform vorliegen. Bevorzugt werden die Applikationsformen Zahnpasta, wobei es sich um eine opake oder um eine gelförmige transparente Zahnpasta handeln kann, Mundwasser und Kaugummi; jedoch ist auch jede andere Applikationsform wie beispielsweise Mundspray, Lutsch- oder Kautablette oder Zahnpulver für diesen Zweck geeignet.

Eine Zahnpasta kann opak oder eine durch Verwendung geeigneter, in ihren Brechungsindices mit dem Brechungsindex des Trägermaterials übereinstimmender Poliermittel hergestellte transparente Zahnpasta sein.

Besonders geeignet als Poliermittel sind Aluminiumoxid, insbesondere in Form seines Trihydrats wie α-Aluminiumoxidtrihydrat, mit einer bevorzugten Korngrößenverteilung zwischen etwa 1 und etwa 20, vorzugsweise etwa 10 μm, und Calciumcarbonat. Es ist jedoch auch möglich, Zahnpasten auf anderer Pastengrundlage einzusetzen, die als Poliermittel beispielsweise Alkalialuminiumsilikate wie solche vom Zeolith-Typ A, beschrieben in den EP-PSen Nr. 2690 und 3023, verschiedene Calciumphosphate wie Dicalciumorthophosphat in Form seines Dihydrats oder wasserfrei, Tricalciumphosphat, Calciumpyrophosphat, unlösliche Alkalimetaphosphate, Siliciumdioxide verschiedener Modifikationen wie Siliciumdioxid-Xerogele, - Hydrogele oder gefällte Siliciumdioxide, oder pulverförmige Kunststoffe wie Polymethylmethacrylat mit einer Korngrößenverteilung zwischen etwa 0,5 und etwa 5 μm enthalten.

Es können selbstverständlich auch Poliermittelgemische aus den genannten Substanzen eingesetzt werden, beispielsweise en Gemisch aus α-Aluminiumoxidhydrat und/oder Calciumcarbonat unsdsynthetischem Zeolith A im Verhältnis von etwa 1:1.

Der Poliermittelanteil in den erfindungsgemäßen Zahnpasten liegt vorzugsweise zwischen etwa 20 und 60 Gew.-% der Gesamtzusammensetzung.

Es ist selbstverständlich möglich, die üblichen in Zahnpasten eingesetzten oberflächenaktiven Verbindungen in Mengen bis zu etwa 2,5 Gew.-% der Gesamtzusammensetzung zu verwenden.

Solche synthetischen oberflächenaktiven Stoffe sind beispielsweise Alkylsulfate, Alkylethersulfate, Olefinsulfonate, Natriumlauroylsarcosinat oder ampholytische, nichtionische oder kationaktive Verbindungen oder auch Seifen wie beispielsweise solche von Laurinsäure, Myristinsäure, Palmitinsäure, Stearinsäure oder Gemischen derselben, beispielsweise Cocosölfettsäuren oder Talgfettsäuren.

Eine Übersicht über in Zahnpasten einsetzbare Zusammensetzungen findet sich, wie überhaupt über sonstige zur Herstellung von Zahnpflegemitteln üblicherweise zum Einsatz gelangende Stoffe und die dabei angewandten Herstellungsverfahren, in dem Handbuch von M.S. Balsam und E. Sagarin, "Cosmetics - Science and Technology", 2nd Ed., Vol. 1, S. 423 bis 533 (1972), auf das hier ausdrücklich Bezug genommen wird.

Gleiches gilt hinsichtlich der in Zahnpasten üblicherweise in Mengen zwischen etwa 10 und etwa 35 Gew.-% zum Einsatz gelangenden Feuchthaltemittel, wie Glycerin, Diole wie 1,4-Butandiol oder 1-2-Propandiol oder Zuckeralkohole wie Sorbit, Mannit oder Xylit und Polyglykole mit niederen Molekulargewichten, ebenso für Verdickungsmittel, deren Mengenanteil in Zahnpasten zwischen etwa 0,25 und etwa 5 Gew.-% der Gesamtzusammensetzung liegt.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum, Carayagummi, Guargummi, Xanthangummi und Irish Moos, synthetische Polyelektrolyte wie die Alkalisalze der Polyacrylsäure sowie anorganische Verdickungsmittel, beispielsweise kolloidales Magnesiumaluminiumsilikat oder disperses Siliciumoxid.

In den erfindungsgemäßen Mitteln zur oralen Hygiene können selbstverständlich auch weitere Wirkstoffe Verwendungfinden. Solche sind insbesondere die bekannten kariesprophylaktischen Fluoride, vorzugsweise in einer solchen Menge, daß die Konzentration an reinem Fluor im Mittel etwa 0,05 bis etwa 1 Gew.-%, vorzugsweise 0,1 bis 0,5 Gew.-% des Mittels beträgt.

Geeignete Fluorverbindungen sind insbesondere die verschiederen Salze der Monofluorphosphorsäure wie Natrium-, Kalium- Lithium-, Calcium- and Aluminiummono- und difluorphosphat sowie verschiedene, Fluor in ionisch gebundener Form enthaltene Fluoride, insbesondere Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsproduke dieser Fluoride untereinander und mit anderen Fluorverbindungen, beispielsweise Alkalimanganfluoride.

Weitere, in den erfindungsgemäßen Mitteln zur oralen Hygiene einsetzbare Stoffe sind Zahnbelag entfernende Substanzen, beispielsweise Zinksalze, Mittel zur Verhinderung von Zahnsteinbildung wie Hydroxyethan-1,1-diphosphonsäure oder Alkylendiaminotetramethylenphosphonsäuren und deren wasserlösliche Salze, Allantoin, Azulen, etc..

Im folgenden werden einige Beispiele gegeben, die das Wesen der vorliegenden Erfindung charakterisieren:

| Beispiel 1 | |
|---|---|
| Zahnpasta | |
| α-Aluminiumoxidtrihydrat (Korngrößenverteilung etwa 1-15 μm) | 58,50 (Gew.-%) |
| Sorbitlösung (70%) | 25,50 |
| Xanthum-Gum | 0,60 |
| Natriummonofluorphosphat | 0,80 |
| Saccharin-Natrium | 0,10 |
| Konservierungsmittel | 0,30 |
| Natriumlaurylsulfat | 0,40 |
| Aromagemisch | 0,10 |
| Kupferaspartat | 0,26 |
| 2,4,4-Trichlor-2-hydroxydiphenylether | 0,05 |
| Wasser | ad 100,00 |

Beispiel 2

__Zahnpasta__

| | |
|---|---|
| Synthetisches Zeolith A ($Na_{12}(AlO_2)_{12}(SiO_2)_{12} \cdot 27 H_2O$) | 24,00 (Gew.-%) |
| Dicalciumorthophosphat | 10,00 |
| Carboxymethylcellulose | 1,20 |
| Natriumlaurylsulfat | 2,00 |
| Glycerin | 6,00 |
| Sorbit | 15,00 |
| Konservierungsmittel | 0,30 |
| Aromagemisch | 1,00 |
| Pyrogene Kieselsäure | 1,55 |
| Saccharin-Natrium | 0,05 |
| Natriummonofluorphosphat | 0,80 |
| Kupferaspartat | 0,25 |
| 2,4,4'-Trichlor-2'-hydroxydiphenylether | 0,08 |
| Wasser | ad 100,00 |


Beispiel 3

__Zahnpasta__

| | |
|---|---|
| Irish Moos | 0,50 (Gew.-%) |
| Xanthan-gum | 0,50 |
| Glycerin | 7,50 |
| Sorbit | 22,00 |
| Kupferformiat $\cdot$ $4H_2O$ | 0,30 |
| Kupferfluorid ($CuF_2$) | 0,25 |
| Natriumlauroylsarcosinat | 1,40 |
| Gehärtetes Melamin-Formaldehyd-Kondensat (mittlerer Teilchendurchmesser 1–10 µm) | 28,50 |
| Titandioxid | 0,50 |
| Saccharin-Natrium | 0,10 |
| Aromagemisch | 1,00 |
| p-Hydroxybenzoesäuremethylester | 0,10 |
| p-Hydroxybenzoesäure-n-propylester | 0,05 |
| 2,4,4'-Trichlor-2'-hydroxydiphenylether | 0,22 |
| Entsalztes Wasser | 37,30 |

Beispiel 4

Zahnpasta

| | |
|---|---|
| Xanthan-gum | 1,20 (Gew.-%) |
| Glycerin | 15,00 |
| Sorbit | 12,00 |
| Kupfersalicylat ($Cu(C_7H_5O_3)_2 \cdot 4H_2O$) | 1,00 |
| 2,4,4'-Trichlorhydroxydiphenylether | 0,12 |
| Siliciumdioxid-Xerogel (vom Typ Syloid[R] AL 1, Oberfläche etwa 800 $m^2$/g) | 16,00 |
| Pyrogenes Siliciumdioxid (vom Typ Aerosil[R]) | 3,00 |
| Titandioxid | 0,50 |
| Aromagemisch | 1,00 |
| Saccharin-Natrium | 0,16 |
| Trinatriumcitrat | 0,25 |
| p-Hydroxybenzoesäuremethylester | 0,20 |
| Entsalztes Wasser | ad 100,00 |

Beispiel 5

Zahnpasta

| | |
|---|---|
| Glycerin | 19,00 (Gew.-%) |
| Sorbit (70%ig) | 7,00 |
| Polyethylenglykol 300 | 3,00 |
| Kupferlactathydrat | 1,20 |
| Zinnfluorid ($SnF_2$) | 0,40 |
| Hydroxyethan-1,1-diphosphonsäure, Trinatriumsalz | 1,25 |
| Bromchlorophen | 0,05 |
| 2,4,4'-Trichlor-2'-hydroxyphenylether | 0,06 |
| Benzoesäure | 0,15 |
| Dehydracetsäure | 0,10 |
| p-Hydroxybenzoesäure-n-propylester | 0,05 |
| Polymethylmethacrylatpulver (mittlerer Teilchendurchmesser 3–8 µm) | 20,00 |
| Siliciumdioxid-Xerogel (vom Typ Syloid[R] 70, Oberfläche etwa 290 $m^2$/g) | 8,50 |
| Pyrogenes Siliciumdioxid (vom Typ Aerosil[R]) | 1,20 |
| Natriumlaurylethersulfat (25%ig in Ethanol) | 10,00 |
| Xanthan-Gum | 0,80 |
| Entsalztes Wasser | ad 100,00 |

**Beispiel 6**

**Kaugummi:**

| | |
|---|---|
| Gummibase | 30,00 (Gew.-%) |
| Sorbit | 25,00 |
| Xylit | 20,00 |
| Saccharin-Natrium | 0,30 |
| Zinkacetat · 2 $H_2O$ | 2,30 |
| 2,4,4'-Trichlor-2'-hydroxydiphenylether | 0,30 |
| Kupfersulfat · 2 $H_2O$ | 0,30 |
| Glycerin | 2,00 |
| Aromagemisch | 3,80 |
| Ascorbinsäure | 1,00 |
| Fructose | 15,00 |

**Beispiel 7**

**Mundwasser-Konzentrat:**

| | |
|---|---|
| Aromagemisch | 5,00 (Gew.-%) |
| Kupferaspartat | 2,50 |
| Zinkcitrat · 2 $H_2O$ | 0,25 |
| 2,4,4'-Trichlor-2'-hydroxydiphenylether | 0,35 |
| Nichtionischer Emulgator | 1,80 |
| n-Propanol | 5,00 |
| 1-Methoxypropanol(-2) | 35,00 |
| Glycerin | 8,50 |
| Phenylsalicylat | 0,55 |
| Saccharin-Natrium | 0,30 |
| Wasser | ad 100,00 |

**Beispiel 8**

**Kaugummi:**

| | |
|---|---|
| Gummibase | 30,00 (Gew.-%) |
| Sorbit | 25,00 |
| Xylit | 20,00 |
| Saccharin-Natrium | 0,30 |
| Kupferfluorid | 0,50 |
| Kupfersulfat · 2 $H_2O$ | 1,30 |
| 2,4,4'-Trichlor-2'-hydroxydiphenylether | 2,00 |
| Glycerin | 2,00 |
| Aromagemisch | 2,70 |
| Ascorbinsäure | 1,00 |
| Fructose | 15,00 |

7

**Patentansprüche**

1. Mittel zur oralen Hygiene mit einem Gehalt an Kupferverbindungen, dadurch gekennzeichnet, daß es zusätzlich 2,4,4'-Trichlor-2'-hydroxydiphenylether enthält.

2. Mittel nach Anspruch 1, dadurch gekennzeichnet, daß es etwa 0,02 bis etwa 1,5 Gew.-%, berechnet auf die Gesamtzusammensetzung, 2,4,4'-Trichlor-2'-hydroxydiphenylether enthält.

3. Mittel nach Anspruch 2, dadurch gekennzeichnet, daß es etwa 0,05 bis etwa 0,5 Gew.-%, berechnet auf die Gesamtzusammensetzung 2,4,4'-Trichlor-2'-hydroxydiphenylether enthält.

**Claims**

1. Composition for oral hygiene containing copper compounds, characterized in that it additionally contains 2,4,4'-trichloro-2'-hydroxydiphenyl ether.

2. Composition according to claim 1, characterized in that it contains from about 0.02 to about 1.5% by weight of 2,4,4'-trichloro-2'-hydroxydiphenyl ether, calculated to the total composition.

3. Composition according to claim 2, characterized in that it contains from about 0.05% to about 0.5% by weight of 2,4,4'-trichloro-2'-hydroxydiphenyl ether, calculated to the total composition.

**Revendications**

1. Composition d'hygiène orale, comprenant une teneur en composés de cuivre, caractérisée en ce qu'elle contient en supplément de l'éther 2,4,4'-trichloro-2'-hydroxydiphénylique.

2. Composition suivant la revendication 1, caractérisée en ce qu'elle contient environ 0,02 à environ 1,5% en poids, par rapport à la composition globale, d'éther 2,4,4'-trichloro-2'-hydroxydiphénylique.

3. Composition suivant la revendication 2, caractérisée en ce qu'elle contient environ 0,05 à environ 0,5% en poids, par rapport à la composition globale, d'éther 2,4,4'-trichloro-2'-hydroxydiphénylique.